# EUROPÄISCHE PATENTSCHRIFT

(11) **EP 3 030 282 B1**
(45) Veröffentlichungstag und Bekanntmachung des Hinweises auf die Patenterteilung: **06.12.2017**
(21) Anmeldenummer: 14750150.6
(22) Anmeldetag: 06.08.2014
(51) Int. Cl.: A61M 1/14, A61M 1/34, B29C 65/16, B23K 26/20, B29C 65/00

(54) **KASSETTENMODUL**
CASSETTE MODULE
MODULE DE TYPE CARTOUCHE

(30) Priorität: 09.08.2013 DE 102013013414
(43) Veröffentlichungstag der Anmeldung: 15.06.2016
(73) Patentinhaber: Fresenius Medical Care Deutschland GmbH, 61352 Bad Homburg (DE)
(72) Erfinder: KREBER, Stefan, 66113 Saarbrücken (DE); WEIS, Manfred, 66606 St. Wendel (DE); WENKE, Marina, 66606 St. Wendel (DE)
(74) Vertreter: Herrmann, Uwe
(86) Internationale Anmeldenummer: PCT/EP2014/002167
(87) Internationale Veröffentlichungsnummer: WO 2015/018524

(56) Entgegenhaltungen:
- US-A1- 2007 278 155
- US-A1- 2009 137 940
- US-A1- 2011 028 902
- US-A1- 2012 167 673

## Beschreibung

Die vorliegende Erfindung betrifft ein Kassettenmodul zur Steuerung von Fluidströmen und zur Verwendung in Blutbehandlungssystemen oder Infusionssystemen.
Aus dem Stand der Technik sind Kassettenmodule beispielsweise für den Einsatz bei Dialysegeräten, insbesondere bei der Hämodialyse oder Peritonealdialyse bekannt. Diese Kassettenmodule haben die Aufgabe, im Rahmen der Behandlung Flüssigkeitsströme, insbesondere Blut, Dialysat oder sonstige behandlungsrelevante Flüssigkeiten mit Hilfe von integrierten Funktionseinheiten steuern bzw. fördern zu können. Solche Funktionseinheiten sind beispielsweise Ventile, Pumpen, Kanäle etc. Durch diese lassen sich die Ströme von Blut, Dialysat und anderen behandlungsrelevanten Flüssigkeiten in der gewünschten Weise steuern.
Kassettenmodule können aufgrund der verwendeten vergleichsweise kostengünstigen Kunststoffe in Massenfertigung hergestellt werden und finden häufig als Einwegartikel Verwendung.
Die genannten Ventil- und Pumpfunktionen sind in Kassettenmodulen aus dem Stand der Technik bekannt. In Kassettenkörpern können Strömungskanäle und/oder Öffnungen ausgebildet sein, die mit einer bewegbaren Membran in Kontakt gebracht werden können, um auf diese Weise die genannte Funktion bereitstellen zu können. So ist es beispielsweise denkbar, die Membran zum Pumpen einer Flüssigkeit zu verwenden sowie auch durch die Membran je nach deren Position eine Öffnung etc. zu überdecken und so eine Ventilfunktion bereitzustellen.

Die Auslenkung der Membran selbst kann beispielsweise durch eine pneumatische Aktuierung erfolgen.

Aus der EP 0 129 554 B1 ist ein Kassettenmodul bekannt, das aus zwei Platten besteht, zwischen denen eine bewegbare Membran angeordnet ist. Diese wird durch eine pneumatische Aktuierung angesteuert. Dazu ist eine der Platten des Kassettenmoduls als Aktorplatte ausgeführt, die mit Kanälen und Kammern ausgeführt ist, in denen je nach Bedarf Überdruck oder Unterdruck erzeugt werden kann.

Die US 5,350,357 offenbart ein Kassettenmodul, das über ein maschinenseitiges pneumatisches Aktormodul betrieben werden kann. Das Kassettenmodul umfasst einen Grundkörper, der auf seiner Vorder- und Rückseite mit einer Membran überzogen ist. Die pneumatische Ansteuerung und damit die Steuerung der Flüssigkeitsströme innerhalb des Kassettenmoduls erfolgt über die pneumatische Auslenkung einer der Membranen.

Die WO 2008/106440 A1 offenbart schließlich eine Kassette, die aus drei Platten besteht, wobei die Flüssigkeitswege innerhalb des Kassettenmoduls im Wesentlichen auf der mittleren Platte angeordnet sind, die zwischen den beiden außenliegenden Platten angeordnet ist. Über einzelne Membranen können die erforderlichen Pump- und Ventilfunktionen erzeugt werden. Die Auslenkung der Membranen erfolgt über pneumatische Anschlüsse, die in einer der Platten angeordnet sind.

Die US 2009/137940 A1 als auch die US2012/167673 A1 zeigen ein Kassettenmodul, das sämtliche Merkmale des Oberbegriffs des Anspruchs 1 aufweist.

Bei aus dem Stand der Technik bekannten Kassettenmodulen ist die zu fördernde Flüssigkeit von der Antriebsseite, d.h. von der Pneumatikseite durch die genannte Membran getrennt.

Kommt es zu einem Riss dieser Membran, führt dies zu einer Kontamination der geförderten Flüssigkeit, wie beispielsweise Blut oder Dialysat mit dem Pneumatikmedium oder zu dem Verlust von Blut oder Dialysat, das durch den Riss auf die Pneumatikseite des Kassettenmoduls strömt.

Der vorliegenden Erfindung liegt somit die Aufgabe zugrunde, ein Kassettenmodul der eingangs genannten Art dahingehend weiterzubilden, dass die Wahrscheinlichkeit für eine Kontamination des in dem Kassettenmodul befindlichen Fluids verringert ist.

Diese Aufgabe wird durch ein Kassettenmodul mit den Merkmalen des Anspruchs 1 gelöst.

Erfindungsgemäß sind somit wenigstens zwei Membranen vorgesehen, die den Fluidstrom (Flüssigkeit oder Gas), der mittels des Kassettenmoduls gesteuert bzw. gefördert werden soll, von einem Aktormedium, wie beispielsweise Druckluft oder einer Flüssigkeit trennt.

Durch den zwischen den Membranen befindlichen Unterdruck wird eine Kopplung der beiden Membranen erreicht und eine unerwünschte Positionierung, wie z.B. das Durchhängen einer der Membranen verhindert. Wird eine Membran durch das Akutatormedium, wie z.B. Druckluft bewegt, wird die andere Membran entsprechend mitgeführt.

Durch die Kopplung der beiden Membranen, bedingt durch das Vakuum, ist sichergestellt, dass die eine Membran unmittelbar der anderen Membran folgt, d. h. beide Membranen in ihrem Bewegungsablauf gekoppelt sind. Ein partieller Verschluss eines Fluidkanals durch eine durchhängende Folie im Unterdruckfall kann somit wirksam vermieden werden.

Die erste Funktionsschicht beinhaltet Mittel, wie z.B. Ventil- oder Pumpmittel, um im Zusammenwirken mit der ersten Membran Strömungsabläufe insbesondere von behandlungsrelevanten Flüssigkeiten zu steuern oder zu regeln.

Das Kassettenmodul kann eine dritte Funktionsschicht aufweisen, die Mittel zur Aktuierung der zweiten Membran aufweist und in dieser Form mit der zweiten Membran zusammenwirkt. Die dritte Funktionsschicht kann beispielsweise Kammern und/oder Strömungskanäle aufweisen, die mit Über- oder Unterdruck beaufschlagbar sind, so dass je nach dem, ob ein Überdruck oder Unterdruck vorliegt, eine Bewegung der zweiten Membran und aufgrund der genannten Kopplung auch der ersten Membran erfolgt. Auf diese Weise ist beispielsweise eine Förderung eines Fluids durch eine Pumpbewegung denkbar oder auch das Verschließen oder Öffnen von Ventilen etc..

Die Begriffe "Überdruck" und "Unterdruck" sind nicht zwingend als Absolutwerte relativ zum Atmosphärendruck zu verstehen, sondern auch als relative Werte.

So bedeutet beispielsweise ein Überdruck oder Unterdruck in einer Kammer nur einen relativen Druck relativ zu der anderen Kammer. So ist es beispielsweise denkbar, dass durch den Betrieb eine Flüssigkeit in eine Kammer einströmt, wodurch in dieser Kammer ein Überdruck relativ zu der anderen Kammer entsteht, ohne dass zwingenderweise in der anderen Kammer Vakuum herrscht.

Gleichwohl ist selbstverständlich auch die Anwendung von Vakuum, d.h. ein Unterdruck relativ zum Atmosphärendruck denkbar und von der Erfindung mit umfasst.

Bei den Mitteln zur Strömungsführung eines Fluidstroms kann es sich um beliebige Mittel handeln, durch die Einfluss auf die Strömung des Fluids genommen werden kann. Dazu gehören beispielsweise ein oder mehrere Kanäle, Ventile oder Pumpen bzw. Ventil- oder Pumpabschnitte.
Die Mittel zur Erzeugung eines Unterdrucks zwischen den Membranen können einen oder mehrere Kanäle umfassen, die mit einer Vakuumpumpe oder dergleichen in Verbindung stehen oder verbindbar sind. So ist es beispielsweise denkbar, dass maschinenseitig eine Vakuumpumpe vorgesehen ist, die derart an das Kassettenmodul angeschlossen ist, dass zwischen den beiden Membranen Vakuum erzeugt werden kann.
Vorzugsweise ist vorgesehen, dass die erste Membran unmittelbar an der ersten Funktionsschicht und an der zweiten Funktionsschicht anliegt und/oder dass die zweite Membran unmittelbar an der zweiten und an der dritten Funktionsschicht anliegt.
Um eine verbesserte Kraftübertragung von einer Membran auf die andere Membran zu erreichen, liegt in bzw. zwischen den beiden Membranen wenigstens ein Mittel zur Verbesserung dieser Kraftübertragung, wie beispielsweise ein Stößel oder eine Stößelscheibe vor.
Weiterhin kann vorgesehen sein, dass der Stößel oder die mit dem Stößel versehene Scheibe derart ausgebildet ist, dass der Stößel bzw. die Scheibe in wenigstens einer Position mit den Mitteln zur Strömungsführung über die erste Membran zusammenwirkt. So ist es beispielsweise denkbar, dass aufgrund des Vorhandenseins des Stößels oder einer Stößelplatte ein Ventil zuverlässig geöffnet oder geschlossen werden kann. Die Dichtfunktion kann von der ersten Membran übernommen werden, die Übertragung der Schließkraft erfolgt zumindest teilweise über die Stößelscheibe.

Weiterhin kann vorgesehen sein, dass das Kassettenmodul, insbesondere die zweite Funktionsschicht wenigstens zwei Randbereiche aufweist, insbesondere an gegenüberliegenden Kanten, in die die Mittel zur Erzeugung eines Unterdrucks zwischen den Membranen münden. Die Mittel zur Erzeugung eines Unterdrucks können beispielsweise durch Kanäle gebildet werden, die sich in der zweiten Funktionsschicht erstrecken und die mit einer Vakuumpumpe oder dergleichen verbunden werden können, so dass zwischen den Membranen ein Unterdruck erzielt werden kann.
Die Funktionsschichten können aus einem biegesteifen Material und die Membranen können aus einem Elastomermaterial bestehen.
Die Membranen sind flexibel, so dass sie einerseits durch das Aktormedium bewegt werden können und andererseits fluidseitig die erforderlichen Auslenkungen vornehmen können.

Die vorliegende Erfindung betrifft des Weiteren eine Blutbehandlungs - oder Infusionsmaschine mit wenigstens einer Aufnahme, in der sich wenigstens ein Kassettenmodul gemäß einem der Ansprüche 1 bis 12 befindet.
Bei der Maschine kann es sich beispielsweise um eine Dialysemaschine oder auch um eine Vorrichtung zur Vornahme einer Infusion handeln. Denkbar ist es, dass es sich bei der Dialysemaschine um eine Hämodialyse oder Peritonealdialysemaschine handelt.
Die Maschine kann Unterdruckmittel, wie beispielsweise eine Vakuumpumpe oder dergleichen aufweisen, die mit den Mitteln zur Erzeugung eines Unterdrucks zwischen den Membranen in Verbindung steht und dort in Betrieb der Maschine einen Unterdruck erzeugt, so dass die Membranen möglichst aneinander liegen und im Falle eines zwischen den Membranen befindlichen Kraftübertragungsmittels möglichst an diesem anliegen.

Ein denkbares Verfahren zur Herstellung des Kassettenmoduls umfasst die Anwendung von Laserstrahlung. Dabei ist es denkbar, dass die Verbindung der Membran zu wenigstens einer weiteren Komponente des Kassettenmoduls mittels Durchstrahlschweißen mit einem Laser erfolgt, wobei vorzugsweise vorgesehen ist, dass die Erwärmung der wenigstens einen das Laserlicht absorbierenden Schicht der Membran erfolgt.

Denkbar ist es, dass die Membran wenigstens eine das Laserlicht transmittierende und wenigstens eine das Laserlicht absorbierende Schicht aufweist, wobei die das Laserlicht absorbierende Schicht den Verbindungsbereich der Membran für wenigstens eine weitere Komponente des Kassettenmoduls bildet. Auch eine einschichtige Ausführung mit einem Absorber für das Laserlicht ist von der Erfindung umfasst.

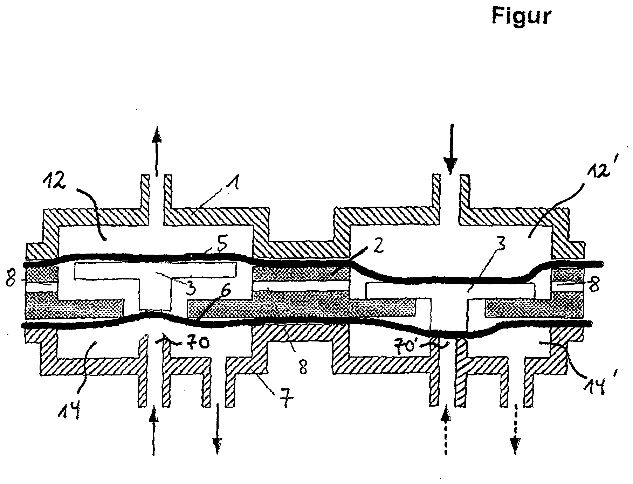

Weitere Einzelheiten und Vorteile der Erfindung werden anhand eines in der Zeichnung dargestellten Ausführungsbeispiels näher erläutert.

Die einzige Figur zeigt eine Längsschnittansicht durch ein erfindungsgemäßes Kassettenmodul.

Mit dem Bezugszeichen 7 ist die erste Funktionsschicht und mit dem Bezugszeichen 2 die zweite Funktionsschicht gekennzeichnet.

Das Bezugszeichen 1 kennzeichnet die dritte Funktionsschicht.

Wie dies aus der Figur hervorgeht, befindet sich die zweite Funktionsschicht 2 zwischen der ersten Funktionsschicht 7 und der dritten Funktionsschicht 1.

Des Weiteren befindet sich eine erste Membran 6 zwischen der zweiten Funktionsschicht 2 und der ersten Funktionsschicht 7 und eine zweite Membran 5 zwischen der dritten Funktionsschicht 1 und der zweiten Funktionsschicht 2.

Das Bezugszeichen 8 kennzeichnet Vakuumkanäle, die sich durch die zweite Funktionsschicht 2 erstrecken und die die Aufgabe haben, ein Vakuum zwischen den beiden Membranen 5, 6 bereitzustellen bzw. aufrechtzuerhalten.

Wie dies aus der Figur weiter hervorgeht, befindet sich zwischen den Membranen 5, 6 in beiden dargestellten Kammern je ein Stößel bzw. je eine Stößelplatte 3, die gleichsam als Ventil zum Verschließen oder Freigeben einer Öffnung 70, 70' in der ersten Funktionsschicht 7 dient.

Im in eine Maschine eingesetzten Zustand des Kassettenmoduls wird durch eine Vakuumpumpe oder dergleichen der Maschine an dem Kanal 8 ein Vakuum angelegt, so dass die beiden Membran 5, 6 eng aneinanderliegen bzw. eng an dem Stößel bzw. an der Stößelplatte 3.

Wie dies weiter aus der Figur hervorgeht, befinden sich in der ersten Funktionsschicht 7 Mittel zur Strömungsführung wenigstens eines Flüssigkeitsstromes, wie beispielsweise von Blut, Dialysat, einer Infusionslösung etc., wobei in dem hier dargestellten Ausführungsbeispiel die Mittel in Form eines Ventils ausgebildet sind, das eine Durchströmung der Kammern 14, 14' des Kassettenmoduls ermöglicht oder verhindert.

Wie dies aus der Figur weiter hervorgeht, weist der Ventilblock bzw. das als Ventilblock ausgeführte Kassettenmodul zwei solcher Ventile auf, wobei das Ventil mit der Öffnung 70 in der links dargestellten Kammer 14 des Kassettenmoduls geöffnet und das Ventil mit der Öffnung 70' in der rechts dargestellten Kammer 14' des Kassettenmoduls geschlossen ist.

Das Öffnen des Ventils erfolgt dadurch, dass in der Pneumatikkammer 12 wie durch einen Pfeil dargestellt Unterdruck erzeugt wird. Dies führt dazu, dass die zweite Membran 5 und aufgrund des zwischen den Membranen herrschenden Vakuums auch die erste Membran 6 sowie auch die Stößelplatte 3 nach oben bewegt wird, wodurch die Öffnung 70 durch die Membran 6 freigegeben wird. Dadurch wird ein Fluidstrom durch die Fluidkammer 14 ermöglicht. Wie bereits oben ausgeführt, bedeuten die Begriffe, Überdruck und Unterdruck nicht zwingenderweise absolute Werte relativ zum Atmosphärendruck, sondern können auch als zueinander relative Werte verstanden werden.

So bedeutet beispielsweise ein Unterdruck in der Kammer 12 nicht nur, dass mit einer Pumpe Vakuum in der Kammer angelegt wird. Es kann auch bedeuten, dass durch den Betrieb der Kassette eine Flüssigkeit in Kammer 14 eingeströmt wird, ohne dass in der Kammer 12 dagegen aktuiert wird.

Die Membranen 5, 6 werden auch in diesem Fall ausgelenkt und die Kammer 12 stellt gegenüber der Kammer 14 die Unterdruckseite dar, ohne dass in der Kammer 12 zwingenderweise Vakuum herrschen muss.

Der Druck in der Funktionsschicht 2 ist geringer als in der Schicht 1 und 14.

In der rechts dargestellten Kammer 12' des Kassettenmoduls wird hingegen ein Überdruck erzeugt, wie dies durch dargestellten Pfeil symbolisiert ist, der das Einströmen eines Pneumatikfluids, insbesondere von Pressluft symbolisiert. Dadurch werden die beiden Membranen 5, 6 sowie auch die rechts dargestellte Stößelplatte 3 nach unten gedrückt und mittels der ersten Membran 6 die Öffnung 70' verschlossen. Die Kammer 14' ist somit nicht von einem Fluid durchströmbar, wie dies durch die gestrichelten Pfeile angedeutet wird.

Bei dem in der Figur dargestellten Kassettenmodul handelt es sich um einen Ventilblock. Grundsätzlich sind auch beliebige andere Funktionalitäten, wie beispielsweise eine Pumpfunktion oder dergleichen von der Erfindung mitumfasst.

Die Funktionsschichten 1, 2, 7 bestehen aus einem biegesteifem Kunststoff, d. h. aus einem formstabilen Kunststoff, wohingegen die Membranen 5, 6 als flexible, vorzugsweise elastische Membranen ausgeführt sind, damit die gewünschte Strömungsführung eingestellt werden kann.

Aus der Figur ist weiter ersichtlich, dass sowohl die erste Funktionsschicht 7 als auch die dritte Funktionsschicht 1 Aufweitungen aufweisen, die Kammern 12, 14, 12', 14' bilden, die einerseits zur Ansteuerung der Membranen über ein Aktormedium, wie z.B. Pressluft und andererseits zur Steuerung eines Fluidstroms, wie beispielsweise von Blut, Dialysat, Infusat etc. dienen.

Das dargestellte Kassettenmodul ist selbstverständlich nicht nur zur Steuerung eines Flüssigkeitsstroms geeignet, sondern auch beispielsweise um einen Gasstrom zu steuern.

Auch die Ansteuerung über die Kammern 12, 12' muss nicht zwingend mit einem Gas bzw. Pressluft erfolgen, sondern kann ebenfalls über eine Flüssigkeit bzw. hydraulisch erfolgen.

Durch den Überdruck bzw. Unterdruck in den Kammern 12, 12' wird das dargestellte Ventil bzw. die Stößelplatte 3 in die ein oder andere Endlage gebracht und verschließt oder öffnet je nach seiner momentanen Position mit seiner der Pneumatik oder Hydraulik entgegengesetzten Seite einen Fluidkreislauf bzw. die Öffnung 70, 70'.

Die Endlagen können beispielsweise durch Flächen der Funktionsschichten, wie beispielsweise durch die zweite Funktionsschicht gebildet werden. In der rechten Kammer steht der plattenförmige Bereich der Stößelplatte an der zweiten Funktionsschicht an, so dass dadurch eine Endlage definiert ist.

Durch das Vorhandensein zweier Membranen 5, 6 wird der Ansteuerungskreislauf, beispielsweise der Pneumatikkreislauf und der Fluidkreislauf, beispielsweise ein Kreislauf zur Förderung von Blut, Dialysat, etc. zuverlässig getrennt, so dass bei dem Reißen einer Membran keine Kontamination auftreten kann.

Aufgrund der Tatsache, dass zwischen den Membranen 5, 6 Vakuum herrscht und die eine Membran somit der Bewegung der anderen Membran folgt, kann ein zuverlässiges Öffnen des Ventils bzw. der Öffnung 70, 70' auch dann erreicht werden, wenn auf der Fluidseite, d. h. auf Seite der Kammer 14, 14' bzw. der Öffnung 70, 70' Unterdruck herrscht. Andernfalls könnte der Fall eintreten, dass bei geschlossenem Ventil und anliegendem Unterdruck der Durchlass 70, 70' durch Anlegen von Unterdruck in der Kammer 12, 12' nicht mehr geöffnet werden könnte.

Vorzugsweise sind die beiden Membranen 5, 6 nur durch das Anlegen eines Unterdrucks miteinander verbunden.

Die Membran 5 steht vorzugsweise unmittelbar mit den Funktionsschichten 1 und 2 und die Membran steht vorzugsweise unmittelbar mit den Funktionsschichten 2 und 7 in Verbindung.

Grundsätzlich ist es auch denkbar, zusätzliche Kopplungsmittel, wie beispielsweise eine Schweißung vorzusehen, über die die Membranen miteinander verbunden sind. Eine bevorzugte Ausgestaltung der Erfindung besteht jedoch darin, vollständig auf die genannte Schweißung zu verzichten.

Vorzugsweise ist wenigstens ein Sensor in den Kanal 8 vorgesehen oder in einem damit in Verbindung stehenden Leitungselement, der den Druck und/oder den Fluß in dem Kanal messen kann. Somit ist es möglich, eine Ruptur entweder der Membran 5 oder der Membran 6 oder beider Membranen in einem frühen Stadium vorzugsweise durch die Maschine erkennen zu können und besonders bevorzugt direkt nach dem Aufrüsten und noch vor Behandlungsbeginn. Eine Ruptur kann jedoch auch über eine veränderliche Leistungsaufnahme des Vakuumpumpenmittels erkannt werden, die mit der Funktionsschicht 2 in Verbindung steht.

Wird beispielsweise festgestellt, dass das gewünschte Vakuum in dem Kanal 8 und damit auch in dem zwischen den Membranen 5, 6 befindlichen Bereichen nicht gehalten werden kann, kann dies darauf zurückgeführt werden, dass eine Ruptur in einer oder beiden der Membranen 5, 6 vorliegt. In diesem Fall ist das vorzugsweise als Disposable ausgeführte Kassettenmodul gegen ein funktionsfähiges Modul auszutauschen.

An dieser Stelle wird darauf hingewiesen, dass der Begriff "Membran" weit zu fassen ist und jedes flächige und bewegbare Material umfasst, das die genannten Funktionen durchführen kann. Die Membranen können beispielsweise als Folien ausgebildet sein.

Sie sind vorzugsweise für das Fluid sowie auch für das Aktormedium undurchlässig. Damit das Vakuum ohne Weiteres gehalten werden kann, sind sie des Weiteren vorzugsweise weitgehend gasundurchlässig.

Weitere Vorteile der Erfindung bestehen darin, dass eine erhöhte Sicherheit der dargestellten Ventilfunktion auch bei etwaigen Schweißfehlern beim Verbinden der Membranen 5, 6 vorliegt, dass kein partieller Verschluss des Fluidkanals durch eine durchhängende Folie im Unterdruckfall vorliegen kann und dass ein vollständiger Entfall des Schweißvorgangs der Folien an den Ventilen möglich ist.

Durch die zwischen den Membranen 5, 6 befindliche Stößelscheibe 3 erfolgt die durch die zweite Membran 5 ausgeübte Bewegung bzw. Ventilfunktion schneller und präziser. Dies lässt höhere Taktraten der Ventilschaltung zu, was wünschenswert ist.

## Patentansprüche

1. Kassettenmodul zur Steuerung von Fluidströmen und zur Verwendung in Blutbehandlungssystemen oder Infusionssystemen, umfassend:
eine erste Funktionsschicht (7), und
eine zweite Funktionsschicht (2), wobei
die erste Funktionsschicht (7) Mittel zur Strömungsführung eines Fluidstroms aufweist,
das Kassettenmodul des Weiteren eine erste Membran (6) und eine zweite Membran (5) aufweist, von denen die erste Membran (6) mit den Mitteln zur Strömungsführung eines Fluidstroms zusammenwirkt, und wobei die zweite Funktionsschicht (2) zwischen den beiden Membranen (5, 6) angeordnet ist und Mittel zur Erzeugung eines Unterdrucks zwischen den Membranen (5, 6) aufweist,
**dadurch gekennzeichnet, dass**
in der ersten Membran (6) und/oder in der zweiten Membran (5) und/oder zwischen den beiden Membranen (5, 6) ein Mittel (3) zur Verbesserung der Kraftübertragung von einer zur anderen Membran (5, 6) vorliegt.

2. Kasssettenmodul nach Anspruch 1, **dadurch gekennzeichnet, dass** das Kassettenmodul eine dritte Funktionsschicht (1) aufweist, die Mittel zur Aktuierung der zweiten Membran (5) aufweist und mit der die zweite Membran zusammenwirkt.

3. Kassettenmodul nach Anspruch 2, **dadurch gekennzeichnet, dass** die es sich bei den Mitteln zur Aktuierung der zweiten Membran (5) um pneumatisch oder hydraulisch wirkende Mittel, insbesondere um Kammern (12, 12') oder Kanäle handelt, in denen Über- oder Unterdruck herrscht oder erzeugbar ist.

4. Kassettenmodul nach einem der vorhergehenden Ansprüche, **dadurch gekennzeichnet, dass** die Mittel zur Strömungsführung eines Fluidstroms einen oder mehrere Kanäle, Ventil- und/oder Pumpabschnitte aufweist.

5. Kassettenmodul nach einem der vorhergehenden Ansprüche, **dadurch gekennzeichnet, dass** die Mittel zur Erzeugung eines Unterdrucks zwischen den Membranen (5, 6) einen oder mehrere Kanäle umfassen oder aus diesen bestehen, die mit einer Vakuumpumpe in Verbindung stehen oder verbindbar sind.

6. Kassettenmodul nach einem der vorhergehenden Ansprüche, **dadurch gekennzeichnet, dass** die erste Membran (6) unmittelbar oder mittelbar an der ersten Funktionsschicht (7) und die erste und die zweite Membran (5, 6) unmittelbar oder mittelbar an der zweiten Funktionsschicht (2) anliegt.

7. Kassettenmodul nach einem der Ansprüche 2 bis 6, **dadurch gekennzeichnet, dass** die zweite Membran (5) unmittelbar oder mittelbar an der zweiten (2) und an der dritten Funktionsschicht (1) anliegt.

8. Kassettenmodul nach Anspruch 1, **dadurch gekennzeichnet, dass** es sich bei dem Mittel (3) um wenigstens einen Stößel oder um wenigstens eine mit einem Stößel versehene Scheibe handelt.

9. Kassettenmodul nach Anspruch 8, **dadurch gekennzeichnet, dass** der Stößel oder die mit einem Stößel versehene Scheibe derart ausgebildet ist, dass dieser/diese in wenigstens einer Position mit den Mitteln zur Strömungsführung eines Fluidstroms zusammenwirkt.

10. Kassettenmodul nach einem der vorhergehenden Ansprüche, **dadurch gekennzeichnet, dass** das Kassettenmodul wenigstens zwei Randbereiche aufweist, insbesondere gegenüber liegende oder benachbart zueinander angeordnete Kanten aufweist, in die die Mittel zur Erzeugung eines Unterdrucks zwischen den Membranen (5, 6) münden und/oder dass das Kassettenmodul wenigstens einen Sensor aufweist, der zur Messung des zwischen den Membranen (5, 6) befindlichen Druckes bestimmt ist.

11. Kassettenmodul nach einem der vorhergehenden Ansprüche, **dadurch gekennzeichnet, dass** wenigstens eine, mehrere oder alle Funktionsschichten (7, 2, 1) aus einem biegesteifen Material, vorzugsweise aus Kunststoff bestehen.

12. Kassettenmodul nach einem der vorhergehenden Ansprüche, **dadurch gekennzeichnet, dass** sich bei der ersten (6) und/oder bei der zweiten Membran (5) um eine elastische Membran handelt.

13. Blutbehandlungs- oder Infusionsgerät mit wenigstens einer Aufnahme, in der sich wenigstens ein Kassettenmodul gemäß einem der Ansprüche 1 bis 12 befindet.

14. Gerät nach Anspruch 13, **dadurch gekennzeichnet, dass** das Gerät Unterdruckmittel aufweist, die mit dem Mitteln zur Erzeugung eines Unterdrucks zwischen den Membranen (5, 6) in Verbindung steht und dort im Betrieb des Geräts einen Unterdruck erzeugt und/oder dass geräteseitig und/oder kassettenmodulseitig wenigstens ein Sensor zur Messung des Druckes zwischen den Membranen (5, 6) oder eines damit korrelierten Parameters vorhanden ist.

## Claims

1. A cassette module for controlling fluid flows and for use in blood treatment systems or in infusion systems, comprising:
a first functional layer (7); and
a second functional layer (2), wherein
the first functional layer (7) has means for the flow guidance of a fluid flow;
the cassette module further comprises a first membrane (6) and a second membrane (5), of which the first membrane (6) cooperates with the means for flow guidance of a fluid flow; and
wherein the second functional layer (2) is arranged between the two membranes (5, 6) and has means for generating an underpressure between the membranes (5, 6),
**characterized in that**
a means (3) for improving the force transmission from one membrane to the other membrane (5, 6) is present in the first membrane (6) und/or in the second membrane (5) und/or between the two membranes (5, 6).

2. A cassette module in accordance with claim 1, **characterized in that** the cassette module has a third functional layer (1) which has means for actuating the second membrane (5) and with which the second membrane cooperates.

3. A cassette module in accordance with claim 2, **characterized in that** the means for actuating the second membrane (5) are pneumatically or hydraulically acting means, in particular chambers (12, 12') or channels, in which excess pressure or underpressure is present or can be generated.

4. A cassette module in accordance with one of the preceding claims, **characterized in that** the means for the flow guidance of a fluid flow has one or more channels, valve sections and/or pump sections.

5. A cassette module in accordance with one of the preceding claims, **characterized in that** the means for generating an underpressure between the membranes (5, 6) comprise or consist of one or more channels which are connected or connectable to a vacuum pump.

6. A cassette module in accordance with one of the preceding claims, **characterized in that** the first membrane (6) is directly or indirectly in contact with the first functional layer (7) and the first and the second membranes (5, 6) are directly or indirectly in contact with the second functional layer (2).

7. A cassette module in accordance with any one of claims 2 to 6, **characterized in that** the second membrane (5) is directly or indirectly in contact with the second (2) or third functional layer (1).

8. A cassette module in accordance with claim 1, **characterized in that** the means (3) is at least one tappet or at least one disk provided with a tappet.

9. A cassette module in accordance with claim 8, **characterized in that** the tappet or the disk provided with a tappet is configured such that it cooperates in at least one position with the means for flow guidance of a fluid flow.

10. A cassette module in accordance with one of the preceding claims, **characterized in that** the cassette module has at least two marginal regions, in particular edges disposed opposite one another or arranged adjacent to one another, into which the means for generating an underpressure open between the membranes (5, 6); and/or **in that** the cassette module has at least one sensor which is intended for measuring the pressure present between the membranes (5, 6).

11. A cassette module in accordance with one of the preceding claims, **characterized in that** at least one, a plurality of or all functional layers (7, 2, 1) comprise flexurally stiff material, preferably plastic.

12. A cassette module in accordance with one of the preceding claims, **characterized in that** the first membrane (6) and/or the second membrane (5) is/are elastic membranes.

13. A blood treatment machine or infusion machine, having at least one slot in which at least one cassette module in accordance with one of the claims 1 to 12 is located.

14. A machine in accordance with claim 13, **characterized in that** the machine has underpressure means which is connected to the means for generating an underpressure between the membranes (5, 6) and generates an underpressure there in the operation of the machine; and/or **in that** at least one sensor is present at the machine side and/or cassette module side for measuring the pressure between the membranes (5, 6) or a parameter correlated therewith.

## Revendications

1. Module de type cartouche destiné à la commande de courants de fluide et à l'utilisation dans des systèmes de traitement de sang ou dans des systèmes de perfusion, comprenant :
une première couche fonctionnelle (7), et
une deuxième couche fonctionnelle (2),
la première couche fonctionnelle (7) présentant des moyens pour le guidage d'écoulement d'un courant de fluide,
le module de type cartouche présentant en outre une première membrane (6) et une deuxième membrane (5), dont la première membrane (6) coopère avec les moyens de guidage d'écoulement d'un courant de fluide, et
la deuxième couche fonctionnelle (2) étant disposée entre les deux membranes (5, 6) et présentant des moyens destinés à générer une dépression entre les membranes (5, 6),
**caractérisé en ce qu'**
un moyen (3) destiné à améliorer la transmission de force d'une membrane à l'autre (5, 6) est présent dans la première membrane (6) et/ou dans la deuxième membrane (5) et/ou entre les deux membranes (5, 6).

2. Module de type cartouche selon la revendication 1, **caractérisé en ce que** le module de type cartouche présente une troisième couche fonctionnelle (1) qui présente des moyens destinés à actionner la deuxième membrane (5) et avec laquelle la deuxième membrane coopère.

3. Module de type cartouche selon la revendication 2, **caractérisé en ce que** les moyens destinés à actionner la deuxième membrane (5) sont des moyens agissant pneumatiquement ou hydrauliquement, notamment des chambres (12, 12') ou des canaux, dans lesquels une surpression ou une dépression règne ou peut être générée.

4. Module de type cartouche selon l'une quelconque des revendications précédentes, **caractérisé en ce que** les moyens de guidage d'écoulement d'un courant de fluide présentent un ou plusieurs canaux, vannes et/ou segments de pompe.

5. Module de type cartouche selon l'une quelconque des revendications précédentes, **caractérisé en ce que** les moyens destinés à générer une dépression entre les membranes (5, 6) comprennent un ou plusieurs canaux ou en sont constitués, lesquels sont en liaison avec une pompe à vide ou peuvent y être reliés.

6. Module de type cartouche selon l'une quelconque des revendications précédentes, **caractérisé en ce que** la première membrane (6) est directement ou indirectement adjacente à la première couche fonctionnelle (7) et **en ce que** la première et la deuxième membranes (5, 6) sont directement ou indirectement adjacentes à la deuxième couche fonctionnelle (2).

7. Module de type cartouche selon l'une quelconque des revendications 2 à 6, **caractérisé en ce que** la deuxième membrane (5) est directement ou indirectement adjacente à la deuxième couche fonctionnelle (2) et à la troisième couche fonctionnelle (1).

8. Module de type cartouche selon la revendication 1, **caractérisé en ce que** le moyen (3) est au moins un coulisseau ou au moins un disque muni d'un coulisseau.

9. Module de type cartouche selon la revendication 8, **caractérisé en ce que** le coulisseau ou le disque muni d'un coulisseau est réalisé de manière à ce que le coulisseau/le disque, dans au moins une position, coopère avec les moyens de guidage d'écoulement d'un courant de fluide.

10. Module de type cartouche selon l'une quelconque des revendications précédentes, **caractérisé en ce que** le module de type cartouche présente au moins deux zones de bord, notamment des arêtes opposées les unes aux autres ou adjacentes les unes aux autres, dans lesquelles débouchent les moyens destinés à générer une dépression entre les membranes (5, 6) et/ou **en ce que** le module de type cartouche présente au moins un capteur qui est destiné à mesurer la pression se trouvant entre les membranes (5, 6).

11. Module de type cartouche selon l'une quelconque des revendications précédentes, **caractérisé en ce qu'**au moins une, plusieurs ou toutes les couches fonctionnelles (7, 2, 1) sont constituées d'une matière résistante à la flexion, de préférence en matière plastique.

12. Module de type cartouche selon l'une quelconque des revendications précédentes, **caractérisé en ce que** la première membrane (6) et/ou la deuxième membrane (5) sont une membrane élastique.

13. Appareil de traitement de sang ou appareil de perfusion comprenant au moins un logement dans lequel se trouve au moins un module de type cartouche selon l'une quelconque des revendications 1 à 12.

14. Appareil selon la revendication 13, **caractérisé en ce que** l'appareil présente des moyens de dépression qui sont en liaison avec les moyens destinés à générer une dépression entre les membranes (5, 6) et y génèrent une dépression lors du fonctionnement de l'appareil et/ou **en ce que** côté appareil et/ou côté module de type cartouche, au moins un capteur est présent pour mesurer la pression entre les membranes (5, 6) ou un paramètre qui y est en corrélation.
